# EUROPÄISCHE PATENTSCHRIFT

(11) **EP 2 109 393 B1**
(45) Veröffentlichungstag und Bekanntmachung des Hinweises auf die Patenterteilung: **08.08.2018**
(21) Anmeldenummer: 08701462.7
(22) Anmeldetag: 14.01.2008
(51) Int. Cl.: A61B 5/145, A61B 5/1455, A61B 5/1459

(54) **OKULARSENSOR ZUM NACHWEIS EINES ANALYTEN IN EINER AUGENFLÜSSIGKEIT**
OCULAR SENSOR FOR THE DETECTION OF AN ANALYTE IN EYE WATER
CAPTEUR OCCULAIRE DESTINÉ À DÉTECTER LA PRÉSENCE D'UN ANALYTE DANS UN LIQUIDE OCCULAIRE

(30) Priorität: 17.01.2007 DE 102007003341
(43) Veröffentlichungstag der Anmeldung: 21.10.2009
(73) Patentinhaber: EyeSense AG, 4051 Basel (CH)
(72) Erfinder: MUELLER, Achim, 63762 Grossostheim (DE)
(74) Vertreter: Herzog, Fiesser & Partner Patentanwälte PartG mbB
(86) Internationale Anmeldenummer: PCT/EP2008/050347
(87) Internationale Veröffentlichungsnummer: WO 2008/087118

(56) Entgegenhaltungen:
- WO-A-01/13783
- AU-A1- 2004 201 752
- US-A1- 2002 099 359
- US-A1- 2004 054 385
- US-B1- 6 312 393
- US-B1- 6 579 690

## Beschreibung

### Gebiet der Erfindung

Die Erfindung betrifft einen Okularsensor zum Nachweis mindestens eines Analyten in einer Augenflüssigkeit, wie beispielsweise in Tränenflüssigkeit, interstitieller Augenflüssigkeit oder Kammerwasser. Weiterhin betrifft die Erfindung ein Messsystem zum Nachweis eines Analyten in der Augenflüssigkeit. Derartige Okularsensoren und Messsysteme werden insbesondere in der medizinischen Diagnostik eingesetzt, beispielsweise zum Nachweis und/oder zur quantitativen Messung einer Glucosekonzentration. Auch andere Anwendungen oder Analyten sind jedoch denkbar.

### Stand der Technik

Die Bestimmung der Blutglukosekonzentration sowie eine entsprechende Medikation ist für Diabetiker ein essentieller Bestandteil des Tagesablaufs. Dabei muss die Blutglukosekonzentration schnell und einfach mehrmals am Tage, typischerweise 2- bis 7-mal bestimmt werden, um gegebenenfalls entsprechende medizinische Maßnahmen ergreifen zu können, wie beispielsweise die Injektion einer angepassten Dosis an Insulin. Neben einer manuellen Injektion erfolgt in vielen Fällen die Medikation auch mittels automatischer Systeme, insbesondere mit Insulinpumpen.

Herkömmliche Systeme zur Bestimmung der Blutglukosekonzentration basieren in der Regel darauf, dass der Patient oder ein Arzt, beispielsweise mittels eines geeigneten Lanzettensystems, einen Hautbereich perforiert und dadurch eine Blutprobe generiert. Diese Probe wird anschließend mittels geeigneter Messverfahren, beispielsweise optischer und/oder elektrochemischer Messverfahren, auf ihren Blutglukosegehalt analysiert. Neben einem Nachweis in Blut kann auch ein Nachweis in anderen Körperflüssigkeiten, wie beispielsweise in Urin erfolgen.

Um die mit der häufigen Generierung von Blutproben verbundenen Unannehmlichkeiten der Patienten zu verringern, wurden verschiedene nicht-invasive oder minimal-invasive Technologien zur Messung von Blutglukosekonzentrationen entwickelt. Eine Technologie basiert auf der Messung von Glukose in Augenflüssigkeiten, wie beispielsweise Tränenflüssigkeit, Kammerwasser oder interstitieller Flüssigkeit. So ist beispielsweise in WO 01/13783 ein Okularsensor für Glukose beschrieben, welcher als Augenlinse ausgestaltet ist. Der Okularsensor umfasst einen Glukoserezeptor, welcher mit einem ersten Fluoreszenzlabel markiert ist, und einen Glukose-Konkurrenten, welcher mit einem zweiten Fluoreszenzlabel ("Donor") markiert ist. Die beiden Fluoreszenzlabel sind derart ausgewählt, dass, wenn der Konkurrent an den Rezeptor gebunden ist, die Fluoreszenz des zweiten Fluoreszenzlabels aufgrund eines resonanten Fluoreszenz-Energietransfers gelöscht wird (Quenching). Durch Überwachen der Veränderung der Fluoreszenzintensität bei einer Wellenlänge um das Fluoreszenzmaximum des quenchbaren Fluoreszenzlabels kann der Anteil des Fluoreszenz-markierten Konkurrenten gemessen werden, welcher durch die Glukose verdrängt worden ist. Auf diese Weise kann die Glukosekonzentration in der Augenflüssigkeit bestimmt werden. Diese Messung kann wiederum genutzt werden, um daraus auf die Blutglukosekonzentration zu schließen. Auch andere Arten von Nachweisen sind denkbar und dem Fachmann geläufig, beispielsweise ein Fluoreszenznachweis des ersten Fluoreszenzlabels.

Auch WO 02/087429 beschreibt ein Fluoreszenz-Photometer, mittels dessen Blutglukosekonzentrationen durch Messung der Glukosekonzentration in einer Augenflüssigkeit bestimmt werden können. Die dargestellte Vorrichtung ist in der Lage, gleichzeitig zwei Fluoreszenzintensitäten bei zwei verschiedenen Wellenlängen zu messen.

Die Messung von Glukose oder anderen Analyten in Augenflüssigkeiten ist üblicherweise durch verschiedene Faktoren limitiert. Ein Faktor besteht beispielsweise darin, dass die Augenflüssigkeiten üblicherweise nur in geringen Mengen zur Verfügung stehen (wie beispielsweise Tränen- oder interstitielle Flüssigkeiten) oder nur schwer zugänglich sind (Glaskörper-Flüssigkeit oder Kammerwasser). Somit stellt in der Regel die Möglichkeit, diese Augenflüssigkeiten als Probe zu sammeln, eine sehr schwierige Vorgehensweise dar. Um diese Einschränkung beziehungsweise Schwierigkeit zu umgehen oder zu verringern, wurden verschiedene Möglichkeiten der in-vivo-Messung entwickelt. Die bereits genannte WO 01/13783 zeigt ein derartiges in-vivo-Messsystem.

Bei der in-vivo Diagnostik hängt das Messsignal häufig nicht nur von der Analyt-Konzentration, sondern auch von der relativen Position des Messgeräts zum Messort ab. Andererseits ist eine mechanisch feste Fixierung des Messgeräts am Patienten nicht möglich und in vielen Fällen auch nicht erwünscht. Einfache mechanische Abstandshalter müssten, um eine hohe Positionsgenauigkeit zu erzielen, auf die individuellen Anforderungen eingestellt werden und sind deshalb nicht für die Massenproduktion geeignet.

Ein weiteres Problem vieler spektroskopischer in-vivo-Messsysteme ist der vergleichsweise geringe spektroskopische Kontrast zwischen Messsignal und Hintergrund. Dies erfordert in vielen Fällen eine aufwändige Kalibrierung, die häufig von der exakten Position abhängt, da der spektrale Hintergrund von der exakten Position abhängt (z. B. aufgrund des unterschiedlichen Streuverhaltens verschiedener Gewebetypen und Blutgefäßarten, der schwankenden Gewebedicke und Gewebedichte etc.). Somit ist bei solchen Messsystemen eine reproduzierbar genaue Positionierung des Messsystems erforderlich.

WO 2004/071287 zeigt ein Fluoreszenz-Photometer, welches mittels zweier verschiedener Strahlen arbeitet und eine korrekte Positionierung des Messgerätes vor dem Auge ermöglicht. Mittels eines Pilotstrahls wird eine erste Fluoreszenz der Pupille angeregt, aus welcher ein Abstand zwischen dem Fluoreszenz-Photometer und dem Auge ermittelt wird. Bei Einstellung eines korrekten Abstandes wird automatisch ein Messstrahl gestartet, welcher im Auge eine zweite Fluoreszenz des Analytsensors anregt, aus welcher wiederum die Analytkonzentration bestimmt werden kann. Trotz des erheblichen Messaufwandes, mit welchem das in WO 2004/071287 gezeigte System verbunden ist, hat sich herausgestellt, dass die Messergebnisse der Analytkonzentration nach wie vor Schwankungen unterworfen sein können. Zudem sind in vielen Fällen selbständig durch den Patienten durchgeführte Positionierungsvorgänge erforderlich, welche gerade von älteren Patienten oder Kindern nur schwer durchführbar sind und welche daher idealerweise vermieden werden sollten.

US2002099359 beschreibt einen Okularsensor, welcher eine Vielzahl von Reservoirs und Sensoren enthält.

### Aufgabe der Erfindung

Aufgabe der Erfindung ist es daher, einen Okularsensor und ein Messsystem bereitzustellen, welche die oben beschriebenen Nachteile und Schwierigkeiten des Standes der Technik vermeiden und eine einfache und sichere Möglichkeit zur Bestimmung der Analytkonzentration in einer Augenflüssigkeit und/oder in einer anderen Körperflüssigkeit, insbesondere in Blut, bereitstellen.

### Beschreibung

Diese Aufgabe wird durch einen Okularsensor gemäß Anspruch 1 und ein Messsystem gemäß Anspruch 13 gelöst. Vorteilhafte Weiterbildungen der Erfindung sind in den Unteransprüchen dargestellt, welche sowohl einzeln als auch in Kombination miteinander realisierbar sind.

Es wird ein Okularsensor zum Nachweis mindestens eines Analyten in einer Augenflüssigkeit vorgeschlagen. Dieser Okularsensor ist dabei derart ausgestaltet, dass dieser in Kontakt mit der Augenflüssigkeit gebracht werden kann, zu welchem Zwecke dieser Okularsensor entsprechend geometrisch ausgestaltet ist und mit geeigneten Materialien hergestellt ist. Besonders bevorzugt ist es, wenn der Okularsensor eine Augenlinse (insbesondere eine neutrale oder korrektive Kontaktlinse) umfasst. Alternativ oder zusätzlich kann der Okularsensor auch ein Augenimplantat umfassen und/oder ein Inlay (z.B. zur Aufnahme im unteren Bindehautsack). In beiden bevorzugten Fällen werden vorzugsweise Materialien eingesetzt, welche biokompatibel sind, das heißt welche nicht toxisch sind und welche bei Einsatz im Auge beziehungsweise bei Implantation im Auge sich nicht auflösen, selbst keinen Schaden nehmen oder toxische Stoffe freisetzen. Bezüglich der Gestaltung einer Augenlinse kann beispielsweise auf WO 01/13783 verwiesen werden. Auch bezüglich der nachzuweisenden Analyten kann beispielsweise auf die Offenbarung dieser Schrift verwiesen werden.

Der erfindungsgemäße Okularsensor weist mindestens ein Sensormaterial auf, welches ausgestaltet ist, um bei Anwesenheit des mindestens einen nachzuweisenden Analyten mindestens eine optische Eigenschaft zu ändern. Bei dieser mindestens einen optischen Eigenschaft kann es sich beispielsweise um eine Farbe handeln, welche sich bei Anwesenheit des Analyten entsprechend ändert. Besonders bevorzugt ist es jedoch, wenn es sich bei der mindestens einen optischen Eigenschaft um eine durch ein Anregungslicht anregbare Lumineszenz, insbesondere eine Fluoreszenz und/oder eine Phosphoreszenz, handelt.

Beispielsweise kann das Sensormaterial ein Material enthalten, welches den nachzuweisenden Analyten binden kann und welches seine Fluoreszenzeigenschaften (zum Beispiel Anregbarkeit, spektrale Eigenschaften oder ähnliches) beim Binden des Analyten ändert. Alternativ oder zusätzlich könnte auch eine spektrale Eigenschaft des Analyten selbst nachgewiesen werden, welche sich beim Binden an die Rezeptoreinheit ändert, beispielsweise infolge eines Quenchings. Wiederum alternativ oder zusätzlich könnte auch eine spektrale Eigenschaft eines weiteren Moleküls nachgewiesen werden, beispielsweise eines Konkurrenzmoleküls, welches an die Rezeptoreinheit des Sensormaterials gebunden ist, bei Anwesenheit des nachzuweisenden Analyten von dieser verdrängt wird und dabei wiederum seine optischen Eigenschaften ändert. Insofern kann sich der Begriff der optischen Eigenschaft auf das Sensormaterial selbst (z.B. einen Rezeptor und/oder ein Konkurrenzmolekül) und/oder den Analyten selbst beziehen, oder auch eine Kombination dieser Stoffe. Verschiedene derartige Sensormaterialien und Nachweisprinzipien sind beispielsweise in WO 01/13783 A1, in WO 02/087429 A1 oder in WO 2004/071287 A1 beschrieben. Auch auf die dort angeführten Materialbeispiele für das Sensormaterial kann beispielhaft verwiesen werden. Dieses Messprinzip kann sowohl zum qualitativen Nachweis als auch zur quantitativen Analyse eingesetzt werden.

Insoweit kann der vorgeschlagene Okularsensor im Wesentlichen den aus dem Stand der Technik bekannten Okularsensoren entsprechen. Im Gegensatz zum Stand der Technik ist erfindungsgemäß der Okularsensor jedoch weiterhin mit mindestens einem Sensorchip versehen. Insbesondere kann es sich bei diesem Sensorchip um einen anwendungsspezifischen integrierten Schaltkreis (application specific integrated circuit, ASIC) handeln, oder der Sensorchip kann einen derartigen ASIC umfassen. Auch andere Arten von Sensorchips sind denkbar, beispielsweise herkömmliche ICs, und/oder der kombinierte Einsatz mehrerer Sensorchips. Auch der Einsatz alternativer Chiptechnologien ist denkbar, beispielsweise der Einsatz organischer Elektronik, wie beispielsweise der Einsatz organischer Transistoren (z.B. Polymertransistoren) und/oder Hybridtechnologien aus organischen und anorganischen Materialien. ASICs können jedoch vorzugsweise auf Grundlage von Silicium-Chips oder anderen Halbleitermaterialien hergestellt sein. Moderne Fertigungsverfahren ermöglichen die Herstellung dünner Sensorchips, beispielsweise Sensorchips mit einer Dicke von 200 bis 400 µm, beispielsweise 250 µm, sowie mit lateralen Abmessungen im Bereich weniger mm. Derartige Chips sind somit problemlos in ein menschliches Auge, beispielsweise in ein Bindehautgewebe, implantierbar und/oder in einer Augenlinse aufnehmbar.

Erfindungsgemäß weist der Sensorchip mindestens einen integrierten optischen Detektor zum Nachweis der optischen Eigenschaft des Sensormaterials auf. Beispielsweise kann der optische Detektor eine oder mehrere Photodioden umfassen, welche ein Lumineszenzlicht des mindestens einen Sensormaterials und/oder des mindestens einen nachzuweisenden Analyten erfassen können. Auch andere Arten von Detektoren sind jedoch einsetzbar, beispielsweise andere Arten von lichtempfindlichen Detektoren ohne Diodencharakteristik.

Besonders bevorzugt ist es dabei, wenn der Okularsensor ein Trägermaterial aufweist, in welches der Sensorchip eingebettet ist. Das Trägermaterial kann beispielsweise durch eine entsprechende Geometrie und/oder Materialauswahl eine Biokompatibilität des Okularsensors gewährleisten, also beispielsweise eine Implantierbarkeit und/oder eine Verwendung in einer oder als Augenlinse. Gleichzeitig kann das Trägermaterial die erforderlichen mechanischen Eigenschaften aufweisen, beispielsweise eine Verformbarkeit und/oder Flexibilität, eine für eine Augenlinse oder ein Implantat geforderte Geometrie oder ähnliches.

In diesem Fall kann das Sensormaterial auf dem Sensorchip aufgebracht werden. Besonders bevorzugt ist es jedoch, wenn das Sensormaterial ganz oder teilweise in dem Trägermaterial enthalten ist. Beispielsweise kann das Sensormaterial in das Trägermaterial eingemischt sein, in diesem gelöst sein, oder ganz oder teilweise Bestandteil dieses Trägermaterials sein (beispielsweise in Form von funktionellen Gruppen, welche an ein Matrixmaterial des Trägermaterials gebunden sind). Auch eine Implementierung in Mikrokapseln ist denkbar, welche dann wiederum beispielsweise in das Trägermaterial eindispergiert sein können. Auch Kombinationen der genannten Techniken sind denkbar.

Das Trägermaterial sollte dabei ein dabei ein für den Analyten zumindest teilweise durchlässiges Material umfassen, beispielsweise ein poröses Material, oder ein Material, welches einen hohen Diffusionskoeffizienten für den nachzuweisenden Analyten aufweist. Insbesondere sollte sichergestellt sein, dass der Analyt in ausreichender Menge in Kontakt mit dem Sensormaterial treten kann. Besonders bevorzugt ist die Verwendung von Hydrogels. Bevorzugt ist es dabei, insbesondere für den Einsatz in einer Augenlinse und/oder in einem Implantat, wenn das Trägermaterial verformbare, insbesondere flexible, Eigenschaften aufweist. Auch eine zumindest teilweise optische Transparenz ist wünschenswert, insbesondere, wenn externes Anregungslicht, wie beispielsweise Tageslicht, für den Analytnachweis genutzt wird (siehe unten).

Durch die Integration des optischen Detektors für den Nachweis der mindestens einen optischen Eigenschaft beziehungsweise Eigenschaftsänderung infolge der Anwesenheit des nachzuweisenden Analyten lassen sich die oben beschriebenen Vorteile realisieren. In weiteren vorteilhaften Ausgestaltungen der Erfindung werden insbesondere Sensormaterialien herangezogen, welche, wie oben beschrieben, eine Lumineszenzeigenschaft in Abhängigkeit von der Anwesenheit des mindestens einen Analyten ändern.

Diese Lumineszenz kann (neben oder alternativ zu Eigenschaften wie beispielsweise Farbe, Brechungsindex etc.) insbesondere eine durch Anregungslicht anregbare Lumineszenz sein, beispielsweise eine Fluoreszenz oder Phosphoreszenz. Die Änderung der optischen Eigenschaft je nach Anwesenheit des Analyten kann dann beispielsweise in einer Zunahme der Fluoreszenz mit steigernder Analytkonzentration liegen. Dies wäre beispielsweise der Fall, wenn eine Fluoreszenz eines Analyt-Rezeptor-Verbundes und/oder eine Fluoreszenz eines durch den Analyten verdrängten, freigesetzten Konkurrenzmoleküls nachgewiesen würde. Alternativ oder zusötzlich kann die Änderung auch in einer Abnahme der Fluoreszenz mit steigernder Analytkonzentration liegen. Letzteres wäre beispielsweise in dem Fall gegeben, in dem ein Fluoreszenzquenching eines Rezeptors bei Anwesenheit des Analyten auftritt und/oder im Falle eines Nachweises der Fluoreszenz eines Rezeptor-Konkurrenzmolekül-Verbundes, wobei das Konkurrenzmolekül durch den Analyten verdrängbar ist. Verschiedene andere Kombinationen und Alternativen der Messung Analyt-sensitiver optischer Eigenschaften sind denkbar.

Im Fall des Fluoreszenz- und/oder Phosphoreszenznachweises können auch eine oder mehrere Anregungslichtquellen zur Erzeugung des Anregungslichts auf dem Sensorchip integriert sein, beispielsweise in Form einer oder mehrerer Leucht- und/oder Laserdioden. Diese Anregungslichtquelle kann, wie auch der mindestens eine optische Detektor, beispielsweise mittels bekannter Techniken hergestellt sein und kann vorzugsweise in einem ASIC integriert sein. Neben der Verwendung herkömmlicher anorganischer Halbleitertechniken können jedoch auch weitere Techniken eingesetzt werden, beispielsweise Techniken, welche auf organische Halbleitertechnik zurückgreifen und beispielsweise organische integrierte Schaltkreise und/oder organische Leuchtdioden und/oder organische Photodetektoren umfassen.

Der Okularsensor ist in diesem Fall derart ausgestaltet, dass dieser eine Anregung des Sensormaterials durch die Anregungslichtquelle ermöglicht. Neben der zuvor genannten Möglichkeit der Integration der Anregungslichtquelle besteht jedoch auch die Möglichkeit, externe Lichtquellen als Anregungslicht zu verwenden. Aufgrund der Verfügbarkeit kann insbesondere Tageslicht als Anregungslicht verwendet werden. Diese Möglichkeit ist besonders einfach zu realisieren, da Tageslicht ein breites Spektrum aufweist und da somit keine interne Energieversorgung für eine Anregungslichtquelle erforderlich ist. Allerdings ist der Begriff des Tageslichts hierbei weit zu fassen, so dass neben natürlichem Tageslicht Umgebungslicht jeglicher Art, wie beispielsweise auch Licht einer oder mehrerer künstlicher Lichtquellen, von diesem Begriff umfasst sein soll. Dadurch kann die Anwendung zu verschiedenen Tageszeiten und unter wechselnden Umgebungsbedingungen ermöglicht werden.

In beiden Fällen, also bei integrierter Erzeugung des Anregungslichts sowie auch bei Verwendung einer externen Anregungslichtquelle, beispielsweise Tageslicht, wird vorzugsweise verhindert, dass das Anregungslicht in den optischen Detektor zum Nachweis der mindestens einen optischen Eigenschaft gelangt. Zu diesem Zweck kann der Okularsensor insbesondere einen optischen Hintergrundfilter aufweisen, insbesondere einen optischen Bandpassfilter oder Kantenfilter, welcher ausgestaltet und angeordnet ist, um eine Intensität des Anregungslichtes ganz oder teilweise zu unterdrücken. Damit kann das Hintergrundsignal, welches der optische Detektor liefert und welches durch das Anregungslicht und nicht durch die Änderung der optischen Eigenschaft des Sensormaterials bedingt ist, stark reduziert werden. Weiterhin kann der Hintergrundfilter aus dem zur Verfügung stehenden Anregungslicht einen vorgegebenen spektralen Bereich auswählen (transmittieren), welcher dann als eigentliches Anregungslicht dient, um das Sensormaterial und/oder ein Referenzmaterial anzuregen.

Verschiedene Filtertechniken sind dem Fachmann bekannt. Beispielsweise können absorptive Filtertechniken eingesetzt werden, beispielsweise einfache Farbfilter. Alternativ oder zusätzlich sind jedoch auch aufwändigere Filtertechniken einsetzbar, beispielsweise die Verwendung von Interferenzfiltern.

Der Hintergrundfilter kann dabei als separater Hintergrundfilter ausgestaltet sein, beispielsweise in Form eines auf dem Sensorchip angeordneten Filterelements oder in Form eines ebenfalls im Trägermaterial eingebetteten Filterelements. Alternativ oder zusätzlich kann der Hintergrundfilter jedoch auch ganz oder teilweise als Bestandteil des Trägermaterials ausgestaltet sein, beispielsweise indem ein als Absorptionsfilter wirkender Farbstoff in das Trägermaterial eingemischt oder in diesem gelöst wird. Wiederum ist jedoch auch eine unmittelbare chemische Implementierung in das Trägermaterial möglich, beispielsweise in Form entsprechender funktioneller Gruppen im Trägermaterial.

Als weitere (alternative oder zusätzliche) Maßnahme zur Verbesserung der Signalqualität des Okularsensors kann ein Sensorfilter vorgesehen sein, welcher ausgestaltet ist, um gezielt die Messung der optischen Eigenschaft begünstigen, andere zum Signal beitragende Anteile jedoch zu unterdrücken. Im Falle einer Verwendung eines lumineszenten Sensormaterials kann beispielsweise der Sensorfilter einen Bandpassfilter und/oder einen Kantenfilter umfassen, welcher ausgestaltet und angeordnet ist, um Lumineszenzlicht des Sensormaterials zu transmittieren (das heißt zumindest teilweise hindurchzulassen, vorzugsweise mit einer Transmission größer als 50 %), wohingegen Licht mit einer Wellenlänge außerhalb des Wellenlängenbereichs der Lumineszenz (das heißt außerhalb eines vorgegebenen Wellenlängenbereichs um das Maximum der Lumineszenz herum) zumindest teilweise unterdrückt wird (d.h. beispielsweise mit einer Transmission von weniger als 50 % transmittiert wird). Dabei lassen sich grundsätzlich die gleichen Filtertechniken einsetzen wie bei dem oben bereits beschriebenen Hintergrundfilter. Diese Weiterbildung der Erfindung hat den Vorteil, dass die Signalqualität (z.B. das Signal-zu-RauschVerhältnis) weiter verbessert wird, da lediglich der einen Informationsgehalt bezüglich des Analyten aufweisende Anteil an Licht gemessen wird.

Weitere vorteilhafte Ausgestaltungen beziehen sich auf die Verwendung von Referenzdetektoren und/oder von Hintergrunddetektoren. So kann beispielsweise eine Referenzeinrichtung vorgesehen sein, wobei der Okularsensor weiterhin ein Referenzmaterial umfasst. Beispielsweise kann dieses Referenzmaterial wiederum als separate Schicht in den Okularsensor eingebracht sein, beispielsweise in Form einer auf dem Sensorchip aufgebrachten Schicht, oder das Referenzmaterial kann in ein Trägermaterial implementiert sein. Bezüglich der Möglichkeiten der Implementierung in das Trägermaterial gilt das oben hinsichtlich des Sensormaterials Gesagte analog.

Das Referenzmaterial sollte dabei derart ausgestaltet sein, dass dieses mindestens eine optische Eigenschaft, insbesondere wiederum eine Lumineszenz (beispielsweise eine Fluoreszenz oder Phosphoreszenz) in Abhängigkeit von der Intensität des Anregungslichts ändert. Im Gegensatz zum Sensormaterial ist dieses Referenzmaterial jedoch derart ausgestaltet, dass diese Änderung der mindestens einen optischen Eigenschaft, beispielsweise wiederum des Fluoreszenzverhaltens, zumindest im Wesentlichen unabhängig ist von der Anwesenheit und/oder Abwesenheit des mindestens einen nachzuweisenden Analyten. Beispielsweise kann das Referenzmaterial derart ausgestaltet sein, dass seine relative Fluoreszenzänderung bei Anwesenheit des Analyten im Vergleich zur relativen Fluoreszenzänderung des Sensormaterials vernachlässigbar klein ist, beispielsweise im Bereich <1/10, <1/100 oder sogar noch geringer für ein Verhältnis der relativen Fluoreszenzänderungen. Beispielsweise kann es sich bei diesem Referenzmaterial wiederum um ein fluoreszent anregbares Material handeln, dessen Fluoreszenz jedoch im Wesentlichen nicht durch den Analyten beeinflusst wird.

In diesem Falle kann der Okularsensor weiterhin mindestens einen optischen Referenzdetektor umfassen, beispielsweise wiederum eine Photodiode. Wiederum ist dieser optische Referenzdetektor vorzugsweise in den Sensorchip integrierbar, wobei das oben hinsichtlich des optischen Detektors Gesagte analog gilt. Dieser optische Referenzdetektor soll ausgestaltet sein, um die optische Eigenschaft des Referenzmaterials zu messen, beispielsweise die Analyt-unabhängige Fluoreszenz des Referenzmaterials.

Um das von diesem optischen Referenzdetektor generierte Referenzsignal zu verbessern und von störenden Anteilen zumindest weitgehend zu befreien, kann weiterhin mindestens ein Referenzfilter vorgesehen sein. Wiederum kann es sich dabei beispielsweise um einen Bandpassfilter und/oder einen Kantenfilter handeln. Dieser Filter soll derart ausgestaltet sein, dass die Lumineszenz des Referenzmaterials zumindest weitgehend (das heißt vorzugsweise mit einer Transmission größer 50 %) transmittiert wird und somit zum Referenzdetektor gelangen kann. Licht mit einer Wellenlänge außerhalb des Wellenlängenbereichs der Referenzlumineszenz (das heißt außerhalb eines um das Referenzlumineszenzmaximum vorgegebenen Bereichs) soll dabei unterdrückt werden. Auf diese Weise ist sichergestellt, dass der Referenzdetektor zumindest im Wesentlichen ein lediglich von der Anregungsintensität des Anregungslichts abhängiges Referenzsignal generiert. Dieses Referenzsignal kann zur Auswertung des Signals des optischen Detektors genutzt werden, beispielsweise indem beide Signale zueinander in ein Verhältnis gesetzt werden, um auf diese Weise beispielsweise eine Analytkonzentration in der Augenflüssigkeit berechnen zu können. Alternativ sind jedoch auch komplexere Auswertungsalgorithmen möglich.

Alternativ oder zusätzlich zur Verwendung eines Referenzdetektors kann weiterhin ein optischer Hintergrunddetektor vorgesehen sein. Beispielsweise kann es sich dabei wiederum um eine im Chip integrierte Photodiode handeln, wobei das oben Gesagte analog gilt. Dieser Hintergrunddetektor soll ausgestaltet sein, um eine Intensität des Anregungslichts zu messen. Vorzugsweise kann es sich bei diesem Anregungslicht um das eigentliche Anregungslicht handeln, mit dem das Sensormaterial und/oder das Referenzmaterial angeregt wird, also beispielsweise ein bereits durch den Hintergrundfilter gefiltertes Umgebungslicht (z.B. Tageslicht). Auch dieses Hintergrundsignal kann wiederum genutzt werden, um eine Konzentration des Analyten zu bestimmen, beispielsweise wiederum, indem ein Verhältnis aus dem Signal des optischen Detektors und dem Hintergrundsignal gebildet wird. Auch komplexere Auswertungsalgorithmen sind jedoch wiederum denkbar, beispielsweise die Nutzung des Anregungslichts, um ein Hintergrundsignal zu eliminieren.

Neben den oben beschriebenen Ausgestaltungen sind weitere vorteilhafte Weiterbildungen des Okularsensors denkbar. So kann beispielsweise eine geometrische Konstruktion vorgesehen sein, welche eine Lichtfalle aufweist. Diese Lichtfalle kann beispielsweise ausgestaltet sein, um eine direkte Transmission von Anregungslicht, insbesondere von ungefiltertem Umgebungslicht, zum optischen Detektor zu unterdrücken, hingegen eine Diffusion von Sensormaterial, beispielsweise angeregten Sensormolekülen, und/oder eine Diffusion des Analyten hin zum optischen Detektor zu ermöglichen. Auf diese Weise lässt sich die Signalqualität weiter verbessern.

Weitere vorteilhafte Ausgestaltungen der Erfindung betreffen die Art und Weise des Auslesens der Information, welche mittels des Okularsensors generierbar ist. So umfasst der Okularsensor vorteilhafter Weise weiterhin mindestens eine Schnittstelle für den Austausch von Informationen mit einer Auswerteeinheit.

Diese Schnittstelle kann auf unterschiedlichste Weise gestaltet sein. Beispielsweise kann der Sensorchip einen Datenspeicher umfassen, in welchem vom Okularsensor generierte Informationen gespeichert sind und welche abgefragt werden können. So kann beispielsweise der Okularsensor als Augenlinse ausgestaltet sein, welche nach Herausnehmen aus dem Auge (es kann sich beispielsweise um eine Einweglinse handeln) in ein entsprechendes Lesegerät eingefügt wird, in welchem (z.B. über entsprechende elektrische Kontakte) beispielsweise bestimmte Kontaktpads auf dem Sensorchip kontaktiert werden, um gespeicherte Informationen abzufragen.

Alternativ oder zusätzlich kann die mindestens eine Schnittstelle jedoch auch eine Schnittstelle zur drahtlosen Datenübertragung umfassen. Hierbei lassen sich insbesondere Infrarot- und/oder Hochfrequenztechniken einsetzen, welche beispielsweise aus der Transpondertechnik bekannt sind. Diese Weiterbildung hat den Vorteil, dass "online" während einer Messung oder kurz nach dieser Messung Informationen abgefragt werden können, welche dann beispielsweise an den Patienten, einen Arzt oder ein weiteres Gerät, beispielsweise einen Computer oder ein Medikationsgerät, übermittelt werden können. Derartige drahtlose Schnittstellen sind mit den zur Verfügung stehenden ASIC-Technologien realisierbar.

Als besonders vorteilhaft hat sich ein System erwiesen, bei welchem der Okularsensor ein kapazitives Element aufweist. An dieses kapazitive Element, welches beispielsweise eine einzelne Platte eines Kondensators umfassen kann, kann mittels der Auswerteeinheit angekoppelt werden, wobei Informationen ausgetauscht werden können, ohne einen physikalischen Kontakt zwischen der Auswerteeinheit und dem Okularsensor herstellen zu müssen. Auf diese Weise lässt sich auch bei im Auge befindlichen Kontaktlinsen und/oder bei Implantaten ein Datenaustausch bequem und sicher ermöglichen.

Beispielsweise kann die Schnittstelle dabei derart ausgestaltet sein, dass das kapazitive Element, gemeinsam mit einem Ohmschen Widerstandselement und/oder einem induktiven Element, einen Schwingkreis bildet, welcher durch die Auswerteeinheit anregbar ist. In diesem Falle kann der optische Detektor vorzugsweise parallel zu dem Ohmschen Widerstandselement und/oder dem optischen Detektor geschaltet werden. Alternativ oder zusätzlich lassen sich auch gegebenenfalls vorhandene Referenzdetektoren und/oder Hintergrunddetektoren entsprechend schalten. Spricht einer dieser Detektoren an, so ändern sich damit die Eigenschaften des Schwingkreises, insbesondere beispielsweise eine Frequenz des Schwingkreises. Diese Frequenzänderung, welche somit vom generierten Signal (Detektorsignal, Referenzsignal, Hintergrundsignal) abhängig ist, kann von der Auswerteeinheit erfasst werden.

Dabei sind verschiedene Ausgestaltungen denkbar. Beispielsweise kann für jeden der Detektoren ein entsprechender Schwingkreis vorgesehen sein, wobei jeder dieser Schwingkreise vorzugsweise eine unterschiedliche Resonanzfrequenz aufweist. Auf diese Weise können von der Auswerteeinheit beispielsweise zeitgleich oder auch zeitversetzt alle Detektoren abgefragt werden. Alternativ kann jedoch auch bereits im Sensorchip eine Vorverarbeitung erfolgen, beispielsweise durch eine entsprechende Quotientenbildung oder ähnliches, so dass mittels der Auswerteeinheit bereits ein bereinigtes Gesamtsignal und/oder ein vorverarbeitetes Signal ausgelesen wird.

Dementsprechend wird neben dem Okularsensor in einer der oben beschriebenen Ausgestaltungen ein Messsystem zum Nachweis des mindestens einen Analyten in der Augenflüssigkeit vorgeschlagen, welches einen Okularsensor gemäß einer der oben beschriebenen Ausgestaltungen sowie weiterhin mindestens eine Auswerteeinheit umfasst, die eingerichtet ist, um mit dem Sensorchip Informationen auszutauschen.

Diese Auswerteeinheit kann vorzugsweise als vom Okularsensor räumlich getrennte Auswerteeinheit ausgestaltet sein und ist vorzugsweise als portables Gerät ausgebildet. Beispielsweise kann es sich dabei um ein Handgerät handeln, mit einer Kantenlänge von vorzugsweise nicht mehr als 15 cm, vorzugsweise von weniger als 10 cm, welches von einem Patienten bequem in einer Tasche oder am Gürtel mitgeführt werden kann.

Beispielsweise kann bei der beschriebenen Ausgestaltung der Schnittstelle des Okularsensors mit einem kapazitiven Element die Auswerteeinheit mit einer Anregungseinheit ausgestattet sein, welche mit der Schnittstelle des Sensorchips einen angeregten Schwingkreis bildet. Dieses Prinzip hat den Vorteil, dass grundsätzlich der Sensorchip keine interne Energiequelle umfassen muss (was jedoch selbstverständlich gleichwohl der Fall sein kann), wodurch insbesondere die Verwendungszeit implantierter Okularsensoren stark gesteigert werden kann. So kann die Anregungseinheit einen Schwingungsgenerator umfassen, dessen Energie induktiv auf das kapazitive Element der Schnittstelle des Okularsensors übertragen wird. Derartige Systeme haben sich in der Praxis für Übertragungsreichweiten von bis zu ca. 1 m als hervorragend geeignet erwiesen, so dass die Auswerteeinheit für eine Auslesung des Sensorchips sogar in einer Tasche mitgeführt werden kann, und nicht vor das Auge gehalten werden muss. Auf diese Weise können beispielsweise automatisierte Messungen durchgeführt werden, ohne dass irgendeine Nutzerhandlung durch den Patienten erforderlich ist. Derartige Messsysteme sind somit besonders benutzerfreundlich insbesondere für ältere Patienten, Kinder sowie behinderte Patienten, wobei die Gefahr von Fehlbedienungen mittels automatisierter Programmabläufe stark vermindert werden kann. Auch eine Interaktion derartiger Messsysteme mit automatischen Medikationssystemen, wie beispielsweise Insulinpumpen, ist denkbar.

Die Auswerteeinheit kann zusätzlich beispielsweise Schnittstellen für eine Interaktion mit einem Benutzer beinhalten, beispielsweise eine Tastatur, Schnittstellen für einen Computer, ein Display oder ähnliches. Weiterhin kann die Auswerteeinheit selbst einen Computer umfassen, beispielsweise einen Mikrocomputer, welcher entsprechend programmtechnisch eingerichtet sein kann. Auch entsprechende Datenspeicher flüchtiger und/oder nicht flüchtiger Art können vorgesehen sein.

So kann die Auswerteeinheit beispielsweise programmtechnisch derart eingerichtet sein, dass diese eine Konzentration des mindestens einen Analyten in der Augenflüssigkeit unter Verwendung der vom Okularsensor gelieferten Signale beziehungsweise Messergebnisse vornimmt. Dieses Ergebnis kann beispielsweise in einen Datenspeicher oder an den Patienten und/oder über eine Schnittstelle an einen Arzt oder eine Datenbank ausgegeben werden.

In vielen Fällen ist jedoch weniger die Konzentration des mindestens einen Analyten in der Augenflüssigkeit von Interesse. Es werden vielmehr häufig Konzentrationen in anderen Körperflüssigkeiten angegeben, beispielsweise Konzentration in Blut und/oder Urin. Glukose wird beispielsweise üblicherweise als Blutglukose angegeben. So kann dementsprechend die Auswerteeinheit weiterhin eingerichtet sein, beispielsweise durch einen entsprechenden Computer mit entsprechender Software, um eine Konzentration des mindestens einen Analyten in einer weiteren Körperflüssigkeit, beispielsweise in Blut, zu berechnen und entsprechend (siehe oben) auszugeben und/oder zu speichern. Zu diesem Zweck kann die Auswerteeinheit beispielsweise Referenztabellen umfassen, welche eine Konzentration des Analyten in der Augenflüssigkeit in Konzentrationen in anderen Flüssigkeiten umwandeln. Alternativ oder zusätzlich können auch Umrechnungsalgorithmen oder Umrechnungskurven herangezogen werden.

Weiterhin hat es sich gezeigt, dass die Genauigkeit der Ergebnisse stark gesteigert werden kann, wenn das Messsystem zusätzlich ein Kalibriersystem umfasst. Dieses Kalibriersystem kann eingesetzt werden, um beispielsweise die Auswertung der Signale des Sensorchips zu verbessern, das heißt, um die Berechnung der Konzentration des Analyten in der Augenflüssigkeit und/oder der weiteren Körperflüssigkeit unabhängiger von natürlichen Streuungen zu gestalten, wie beispielsweise Abweichungen von Patient zu Patient hinsichtlich der physiologischen Rahmenbedingungen, Abweichungen hinsichtlich der Positionierung des Okularsensors im Auge, Herstellungstoleranzen des Okularsensors oder ähnliches.

Dieses Kalibriersystem kann dementsprechend ausgestaltet sein, um mindestens eine Kalibrationsinformation über eine Konzentration des Analyten in der Augenflüssigkeit und/oder einer weiteren Körperflüssigkeit zu empfangen und/oder zu verarbeiten und dementsprechend eine Kalibration der Bestimmung der Analytkonzentration durchzuführen. Beispielsweise kann das Kalibrationssystem zu diesem Zweck über eine Schnittstelle externe Daten hinsichtlich einer Analytkonzentration in der Augenflüssigkeit und/oder der weiteren Körperflüssigkeit empfangen, welche mittels eines separaten Messsystems gewonnen wurden. Alternativ oder zusätzlich kann das Kalibrationssystem auch selbst mindestens ein Messgerät zur Bestimmung der Konzentration des Analyten umfassen. Besonders bevorzugt sind hierbei konventionelle Messgeräte, beispielsweise Messgeräte, welche die Analytkonzentration mittels eines Testelements bestimmen. Hierbei können beispielsweise elektrochemische Teststreifen und/oder optische Teststreifensysteme, wie sie dem Fachmann beispielsweise aus dem Bereich der Blutglukosebestimmung bekannt sind, eingesetzt werden.

Dabei kann das Messsystem beispielsweise derart ausgestaltet sein, dass vor Inbetriebnahme des Messsystems eine derartige Kalibrationsmessung durchgeführt wird, um die vom Okularsensor gelieferten Daten mit den Daten des "konventionellen" Messgerätes abzugleichen. Weiterhin kann, alternativ oder zusätzlich, auch eine Kalibrationsmessung in regelmäßigen Abständen durchgeführt werden, wobei beispielsweise das Messsystem derart ausgestaltet sein kann, dass dieses einen Benutzer in bestimmten Zeitabständen auf die Notwendigkeit einer derartigen Kalibrationsmessung hinweist (beispielsweise einmal am Tag, im Vergleich zu herkömmlich bis zu siebenmal am Tag erforderlichen "konventionellen" Messungen). Auch automatische Kalibrationsmessungen sind möglich, beispielsweise Kalibrationsmessungen mittels eines weiteren implantierten Sensors.

Insgesamt bewirkt diese Ausgestaltung des Messsystems unter Verwendung eines Kalibriersystems eine weitere Verbesserung der Messgenauigkeit und somit eine Verbesserung der Zuverlässigkeit der gewonnenen physiologischen Informationen über den Patienten.

### Ausführungsbeispiele

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigt:
- Figur 1: eine schematische Darstellung eines Okularsensors;
- Figur 2: eine Darstellung der Filtercharakteristiken der im Okularsensor gemäß Figur 1 verwendeten Filter;
- Figur 3: eine spektrale Darstellung der Lichtverhältnisse im Okularsensor gemäß Figur 1;
- Figur 4: eine schematische Darstellung eines Zusammenwirkens einer Anregungseinheit einer Auswerteeinheit mit einer Schnittstelle des Okularsensors; und
- Figur 5: ein Ausführungsbeispiel eines Messsystems mit einem Okularsensor, einer Auswerteeinheit und einem Kalibrationssystem.

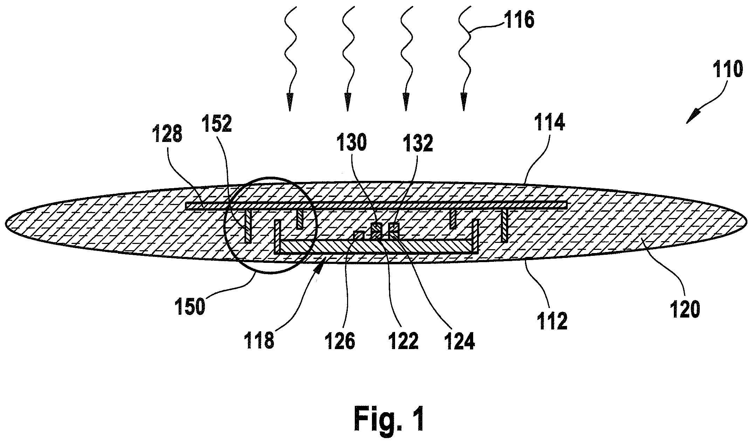

In Figur 1 ist ein Ausführungsbeispiel eines erfindungsgemäßen Okularsensors 110 stark schematisiert dargestellt. Der Okularsensor ist in diesem Fall beispielsweise als Implantat ausgestaltet und kann beispielsweise in oder unter eine Bindehaut eines Patienten implantiert werden. Der Okularsensor 110 weist eine Augenseite 112 und eine Außenseite 114 auf. Dementsprechend kann Tageslicht 116 von der Außenseite 114 her auf den Okularsensor 110 auftreffen.

Der Okularsensor 110 weist einen Sensorchip 118 auf, welcher, wie oben beschrieben, vorzugsweise als ASIC ausgestaltet ist. Dieser Sensorchip 118 ist in ein biokompatibles Hydrogel als Trägermaterial 120 eingebettet. Dieses Trägermaterial verleiht dem Okularsensor 110 damit die erforderliche mechanische Stabilität, ist jedoch gleichzeitig verformbar beziehungsweise flexibel, um sich dem Auge anzupassen und ermöglicht eine Diffusion des Analyten.

In das Trägermaterial 120 ist in diesem Ausführungsbeispiel ein Sensormaterial eingemischt. Beispielsweise kann es sich bei diesem Sensormaterial um Sensormaterialien zum Nachweis von Glukose in einer Augenflüssigkeit, beispielsweise in Tränenflüssigkeit, Kammerwasser oder interstitieller Augenflüssigkeit handeln. Beispiele derartiger Sensormaterialien sind in WO 01/13783 A1, WO 02/087429 A1 und WO 2004/071287 A1 beschrieben. Besonders bevorzugt werden im vorliegenden Ausführungsbeispiel folgende Sensormaterialien eingesetzt: Concanavalin A / Dextran, Glucose-Galaktose bindendes Protein (GGBP), Glukose-Hexokinase Borsäureester (wie beispielsweise in PCT/EP2004/008825 beschrieben). Auch andere Sensormaterialien sind jedoch einsetzbar, sowie auch Mischungen oder Kombinationen mehrerer Sensormaterialien. Dieses Sensormaterial kann direkt im Hydrogel homogen verteilt sein, kann jedoch auch in Mikrokapseln eingeschlossen sein, welche ihrerseits wiederum vorzugsweise im Hydrogel verteilt sind.

Normalerweise wird ein derartiger implantierter Okularsensor 110, wie beispielsweise in WO 01/13783 A1 beschrieben, von außen her, das heißt von der Außenseite 114, mit einer geeigneten Lichtquelle (zum Beispiel einer Leuchtdiode mit einem Bandpassfilter und/oder einer Laserdiode) zur Fluoreszenz angeregt und die Fluoreszenz bei einer oder mehreren Wellenlängen mit einem geeigneten Photometer gemessen. Die Stärke des Fluoreszenzsignals ist dabei naturgemäß jedoch nicht nur von der Analytkonzentration, sondern auch vom Abstand und Winkel zwischen Implantat und Photometer und/oder Anregungslichtquelle abhängig.

Der vorliegende Okularsensor 110 löst diese Problematik, wie oben beschrieben, dadurch, dass dieser den Sensorchip 118 als integralen Bestandteil beinhaltet. Dieser Sensorchip 118, welcher vorzugsweise als ASIC ausgestaltet ist, kann beispielsweise kundenspezifisch gefertigt sein und beispielsweise auf einem organischen oder anorganischen Halbleitermaterial basieren, beispielsweise Silizium. Dabei kann zumindest ein Teil einer Auswerte- und Ansteuerelektronik auf dem Sensorchip 118 integriert sein.

In diesem Ausführungsbeispiel umfasst der Sensorchip 118 drei Photodioden: Neben der eigentlichen Messdiode 122 als optischer Detektor sind eine Referenzdiode 124 und eine Hintergrunddiode 126 auf dem Sensorchip 118 integriert. Die erforderlichen Ansteuer- beziehungsweise Auswerteschaltungen sind ebenfalls auf dem Sensorchip 118 integriert, sind jedoch in Figur 1 nicht dargestellt.

Neben dem Sensormaterial ist in das Trägermaterial 120 weiterhin ein Referenzmaterial eingemischt, beispielsweise ein Referenzfluorophor, dessen Lumineszenz ebenfalls durch das Tageslicht 116 anregbar ist, wobei diese Referenzfluoreszenz jedoch unabhängig von der Anwesenheit des nachzuweisenden Analyten ist.

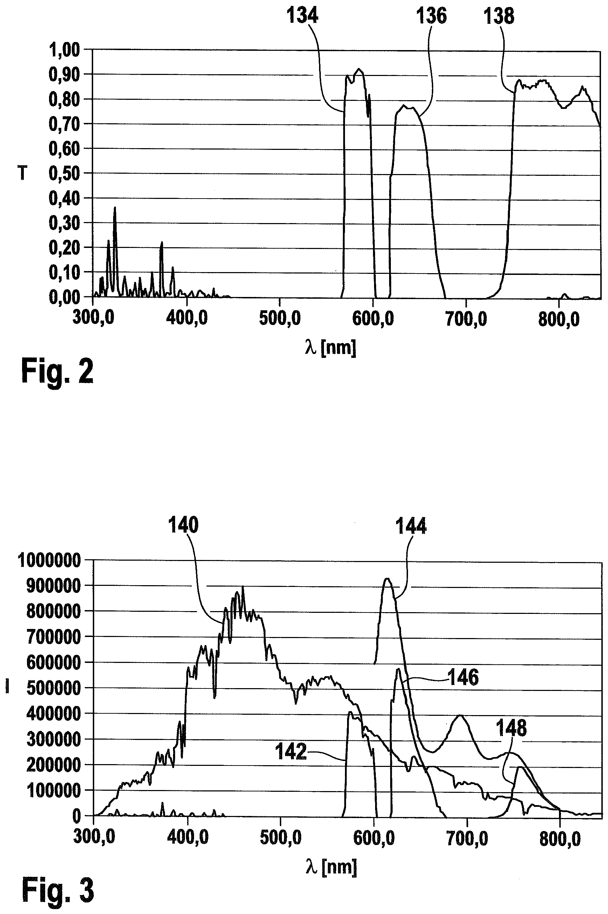

Um die verschiedenen Lichtanteile separat mittels der drei Dioden 122, 124, 126 aufnehmen zu können, sind weiterhin verschiedene Filter vorgesehen. So ist der Sensorchip 118 zunächst mit einem Hintergrundfilter 128 abgedeckt. Dieser Hintergrundfilter 128 ist in diesem Ausführungsbeispiel als Interferenzfilter ausgestaltet. Weiterhin ist die Messdiode 122 mit einem Sensorfilter 130 bedeckt, und die Referenzdiode 124 mit einem Referenzfilter 132. Auch Sensorfilter 130 und Referenzfilter 132 sind vorzugsweise als Interferenzfilter ausgestaltet. Die Hintergrunddiode 126 ist (außer durch den Hintergrundfilter 128) nicht weiter mit einem Filter ausgestattet. Für die Transmissionscharakteristika dieser Filter 128, 130 und 132 wird Bezug genommen auf Figur 2. In Figur 2 sind die Transmissionsspektren (dargestellt ist jeweils eine normierte Transmission T gegen die Wellenlänge λ) dieser Filter 128, 130, 132 aufgetragen. Dabei zeigt die Kurve 134 die Transmission des Hintergrundfilters 128, die Kurve 136 die Transmission des Sensorfilters 130 und die Kurve 138 die Transmission des Referenzfilters 132. Dabei ist zu erkennen, das die Filter 128 und 130 als Bandpassfilter ausgestaltet sind, mit einer Transmission von ca. 560 bis 600 nm beziehungsweise ca. 620 bis 670 nm. Der Referenzfilter 132 ist hingegen im Wesentlichen als Kantenfilter ausgestaltet und "öffnet" ab einer Wellenlänge von ca. 740 nm.

Die drei Filter 128, 130 und 132 sind dabei in ihren spektralen Eigenschaften derart ausgelegt, dass der Filter 128 eine Transmission im Bereich der Anregungswellenlänge des Sensormaterials aufweist. Die Filter 130 und 132 weisen hingegen eine Transmission im Bereich der Lumineszenzwellenlänge des Sensormaterials (Sensorfilter 130) beziehungsweise im Bereich der Fluoreszenz des Referenzmaterials (Referenzfilter 132) auf.

Der als Implantat ausgebildete Okularsensor 110 ist erfindungsgemäß unter der Bindehaut des Auges implantiert, wo er dem normalen Tageslicht 116 ausgesetzt ist. Da die Bindehaut hochgradig transparent ist, dringt, im Unterschied zu normal pigmentierter Haut, ein vergleichsweise hoher Lichtanteil in den als Implantat ausgebildeten Okularsensor 110 ein.

In Figur 3 sind die spektralen Lichtverhältnisse (aufgetragen ist die Intensität I gegen die Wellenlänge λ) im Okularsensor 110 aufgetragen. Dabei zeigt die Kurve 140 die Intensitätsverteilung des Tageslichts 116.

Da der Sensorchip 118 vom Hintergrundfilter 128 (vergleiche die Transmissionscharakteristik 134 in Figur 2) vollständig umgeben ist, dringt vom Tageslicht 116 nur noch der Anteil durch, der für die Anregung des Sensormaterials erforderlich ist. Die spektrale Intensitätsverteilung dieses eigentlichen Anregungslichts ist in Figur 3 mit der Kurve 142 bezeichnet und ergibt sich aus einer Multiplikation der Kurve 134 in Figur 2 mit der Intensitätsverteilung des Tageslichts 140 in Figur 3. Diese Intensität 142 des Anregungslichts wird mit der Hintergrunddiode 126 (siehe Figur 1) auf dem Sensorchip 118 gemessen.

Mit diesem Anregungslicht 142 wird sowohl die Fluoreszenz des Sensormaterials angeregt (Fluoreszenz analytabhängig) als auch die Fluoreszenz des Referenzmaterials (Fluoreszenz analytunabhängig). Somit ergibt sich infolge dieser Anregung eine Gesamtfluoreszenz 144, welche sich aus diesen beiden Fluoreszenzanteilen zusammensetzt.

Zur Trennung dieser Fluoreszenzanteile werden die Messdiode 122 mit dem Sensorfilter 130 und die Referenzdiode 124 mit dem Referenzfilter 132 (vergleiche Figur 1) eingesetzt. Dementsprechend ist die Gesamtfluoreszenz 144 in Figur 3 wiederum mit den Transmissionskurven 136 beziehungsweise 138 gemäß Figur 2 zu multiplizieren. Diese Filterung führt somit zu einer Sensorfluoreszenz 146, welche von der Messdiode 122 erfasst wird, sowie zu einer Referenzfluoreszenz 148, welche von der Referenzdiode 124 erfasst wird. Auf diese Weise liefert die Hintergrunddiode 146 eine Information darüber, wie stark das Sensormaterial und das Referenzmaterial angeregt werden, die Messdiode 122 eine Information über die analytabhängige Sensorfluoreszenz des Sensormaterials und die Referenzdiode 124 eine analytunabhängige Information über die Referenzfluoreszenz des Referenzmaterials. Aus diesen drei Signalanteilen kann mit hoher Genauigkeit auf eine Konzentration des Analyten in der Augenflüssigkeit zurückgeschlossen werden.

Da zwischen den drei Photodioden 122, 124 und 126 und dem Sensormaterial beziehungsweise Referenzmaterial ein fester Verbund besteht, ist das Messsignal beziehungsweise die Messsignale nicht mehr positionsabhängig. Das Messsignal ist somit im Idealfall lediglich von der Anregungsenergie (diese Information liefert die Hintergrunddiode 126) und der Analytkonzentration abhängig. Die gleichzeitige Zurverfügungstellung von Informationen der Hintergrunddiode 126 und der Referenzdiode 124 ist in gewisser Hinsicht redundant, die zusätzlichen Informationen erhöhen jedoch die Robustheit und Messgenauigkeit des Systems. So können beispielsweise, insbesondere wenn das Referenzmaterial und das Sensormaterial ähnliche spektrale Anregungseigenschaften aufweisen, Änderungen in der spektralen Charakteristik dieses Anregungslichtes ausgeglichen werden, wie beispielsweise der Wechsel von Tageslicht auf künstliches Umgebungslicht. Auf dieses Weise wird die Flexibilität der Anwendung erheblich gesteigert, so dass Messungen der Analytkonzentration zu verschiedenen Tageszeiten und/oder unter Wechsel der Lichtverhältnisse (Tageslicht, Kunstlicht) vorgenommen werden können.

Die Anordnung des Okularsensors 110 gemäß Figur 1 weist eine weitere Besonderheit in Form einer Lichtfalle 150 auf. Diese Lichtfalle 150 trägt dem Gedanken Rechnung, dass einerseits die drei Photodioden 122, 124 und 126 auf dem Sensorchip 118 vollständig vom Hintergrundfilter 128 umgeben sein sollten, da sonst eintretendes Tageslicht 116 zu einem Offset führen würde, welcher in der Regel erheblich größer wäre als das eigentliche Messsignal. Andererseits sollte innerhalb des Okularsensors 110 das Sensormaterial aufgenommen sein, und es sollte eine freie Diffusion des Analyten möglich sein. Diese Problematik wird in dem dargestellten Ausführungsbeispiel durch die Lichtfalle 150 gelöst, welche eine Diffusion des Analyten im Trägermaterial 120 ermöglicht, jedoch ein Eindringen von ungefiltertem Tageslicht 116 unterdrückt. Mechanische Lichtfallen 150 werden typischerweise, wie in Figur 1 gezeigt, durch mehrere, sich hinterschneidende Stege 152 realisiert. Auch andere Arten von Lichtfallen 150, wie beispielsweise optische "Labyrinthe", wie sie z.B. aus der Technik der Rauchmelder bekannt sind, sind einsetzbar.

Eine weitere, alternativ oder zusätzlich einsetzbare Möglichkeit besteht darin, das Hydrogel des Trägermaterials 120 selbst mit entsprechenden Farbstoffmolekülen zu markieren, so dass der Hintergrundfilter 128 nicht in Form eines Interferenzfilters, sondern in Form eines Bulkfilters ausgestaltet ist. Auch auf diese Weise lässt sich näherungsweise die Transmission 134 realisieren. Allerdings sind typischerweise Filtercharakteristika derartiger Farbstoffmoleküle breiter als die Filtercharakteristik der Kurve 134 gemäß Figur 2. Weiterhin besteht der Vorteil eines Interferenzfilters darin, dass die spektrale Charakteristik vergleichsweise einfach beeinflusst werden kann. Interferenzfilter könnten beispielsweise durch Abscheidung einer dünnen Metall- und/oder Metallverbindungs-Schichtfolge auf einem transparenten Träger, wie beispielsweise einem dünnen Glas und/oder einem Kunststoffmaterial, hergestellt werden, welches anschließend gemäß Figur 1 in den Okularsensor 110 implementiert wird beziehungsweise auf den Sensorchip 118 aufgesetzt wird. Auch eine direkte Aufbringung der Filter auf den Sensorchip 118, beispielsweise als Folge einer oder mehrerer Aufdampfschichten, ist jedoch möglich, sowie auch eine Implementierung mechanischer Lichtfallen 150 in Form von MEMS (micro electro-mechanical systems) auf dem Sensorchip 118.

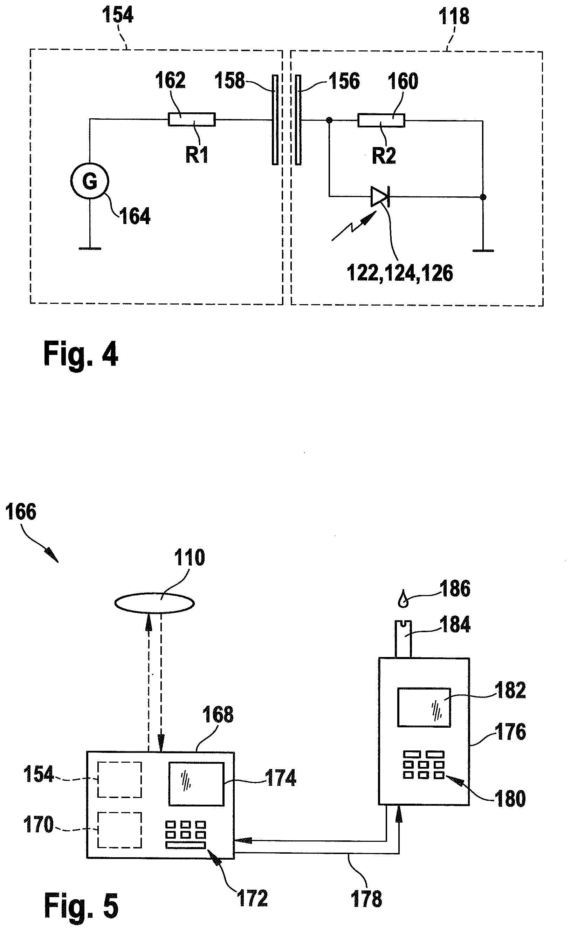

In Figur 4 ist stark schematisiert eine Möglichkeit zum Auslesen des Sensorchips 118 dargestellt. Der Sensorchip 118, welcher hierbei in Figur 4 nur unvollständig wiedergegeben ist, wirkt dabei mit einer Anregungseinheit 154 zusammen, welche in einer Auswerteeinheit (vergleiche Figur 5) implementiert ist. Als Schnittstelle zwischen Sensorchip 118 und Anregungseinheit 154 dienen dabei kapazitive Elemente 156 beziehungsweise 158, welche in Figur 4 schematisch als einzelne Kondensatorplatten dargestellt sind. Dabei ist das kapazitive Element 156 des Sensorchips 118 über einen Ohmschen Widerstand 160 mit Masse (beispielsweise einer größeren Metallfläche auf dem Sensorchip 118) verbunden. Parallel zum Widerstand 160 ist beziehungsweise sind die Dioden 122, 124 und 126 geschaltet. Wie oben dargelegt, kann die Schaltung derart erfolgen, dass diese Dioden einzeln oder alle in der in Figur 4 dargestellten Weise geschaltet werden.

In der Anregungseinheit 154 ist das kapazitive Element 158 über einen Widerstand 162 mit einem Generator 164 verbunden, welcher wiederum an seinem anderen Anschluss mit Masse verbunden ist. Die Anregungseinheit 154 und der Sensorchip 118 bilden bei dieser Beschaltung einen angeregten elektrischen Schwingkreis. Der Generator 164 erzeugt ein sich änderndes elektrisches Feld an den kapazitiven Elementen 156, 158. Die Eigenfrequenz des Schwingkreises wird von den Kapazitäten (bestimmt durch die Zusammenwirkung der kapazitiven Elemente 156, 158) und die Widerstände 160, 162 im Schwingkreis bestimmt. Durch Einstrahlung von Licht auf die Dioden 122, 124, 126 ändert sich der Gesamtwiderstand der Parallelschaltung aus Widerstand 160 und Diode 122, 124, 126 und somit die Eigenfrequenz des Schwingkreises. Diese Änderung kann beispielsweise als Feldstärkeänderung gemessen und ausgewertet werden. Die Anregungseinheit 154 kann dementsprechend eine Feldnadel und eine Feldstärke-Messvorrichtung umfassen, um diese Feldstärkeänderungen zu messen. Alternativ oder zusätzlich können jedoch auch andere Messtechniken eingesetzt werden, wie beispielsweise eine Messung der Leistungsabgabe des Generators 164.

In Figur 5 ist stark schematisiert ein Messsystem 166 zum Nachweis des mindestens einen Analyten in einer Augenflüssigkeit dargestellt. Das Messsystem 166 umfasst einen Okularsensor 110, beispielsweise einen Okularsensor gemäß der in Figur 1 beschriebenen Ausgestaltung, welcher beispielsweise als Implantat und/oder als Augenlinse ausgestaltet sein kann. Weiterhin umfasst das Messsystem 166 eine Auswerteeinheit 168 mit einer Anregungseinheit 154 (beispielsweise gemäß dem Ausführungsbeispiel in Figur 4) und einem Mikrocomputer 170. Die Auswerteeinheit 168 kann beispielsweise über den anhand von Figur 4 beschriebenen Vorgang Informationen mit dem Okularsensor 110 beziehungsweise dem Sensorchip 118 austauschen und kann somit "in situ" (das heißt ohne den Okularsensor 110 aus der Augenflüssigkeit entfernen zu müssen) Messdaten abfragen. Die Auswerteeinheit 168 kann dabei an einer beliebigen Stelle des Körpers des Patienten getragen werden. Der Körper kann dann als Teil der Schnittstelle dienen, zusätzlich zu den kapazitiven Elementen 156, 158 gemäß Figur 4, und trägt zur Datenübertragung bei.

Der Mikrocomputer 170 der Auswerteeinheit 168 ist vorzugsweise gemäß der obigen Beschreibung ausgestaltet und dient zur Steuerung der Messung sowie zur Auswertung der Messergebnisse. Der Mikrocomputer 170 kann durch Bedienelemente 172 von einem Benutzer bedient werden, und über ein Display 174 lassen sich Informationen an einen Benutzer ausgeben. Auch andere Benutzerschnittstellen sind jedoch implementierbar.

Weiterhin umfasst das Messsystem 166 gemäß dem Ausführungsbeispiel in Figur 5 ein Kalibrationssystem 176. Dieses Kalibrationssystem 176 ist vorzugsweise in die Auswerteeinheit 168 implementiert. Bei dem in Figur 5 dargestellten, schematisierten Ausführungsbeispiel ist die Kalibrationseinheit 176 jedoch als separate Einheit ausgebildet, welche über eine Schnittstelle 178 mit der Auswerteeinheit 168 kommuniziert. Dabei kann es sich um eine drahtlose und/oder auch eine drahtgebundene Schnittstelle handeln, oder es können auch Datenaustauschvorrichtungen über manuell vom Patienten zwischen dem Kalibrationssystem 176 und der Auswerteeinheit 168 ausgetauschte Datenträger (z.B. Speicherchips) als "Schnittstellen" zur Verbindung dieser Elemente vorgesehen sein.

Das Kalibrationssystem 176 umfasst wiederum Bedienelemente 180, ein Display 182 sowie (in Figur 5 nicht dargestellt) eine entsprechende Elektronik, beispielsweise wiederum einen Mikrocomputer und/oder andere elektronische Bauelemente. Das Kalibrationssystem 176 ist dabei ausgestaltet, um mittels eines Teststreifens 184 eine Konzentration des Analyten in einer Körperflüssigkeit nachzuweisen, welche hier symbolisch als Blutstropfen 186 dargestellt ist. Somit können als Kalibrationssystem 176 herkömmliche, kommerziell erhältliche Messgeräte, wie beispielsweise Blutglukosemeter, eingesetzt werden. Derartige Systeme verfügen üblicherweise auch über entsprechende Schnittstellen, wie beispielsweise Infrarotschnittstellen. Die mittels des Kalibrationssystems 176 gewonnenen Informationen über die Analytkonzentration in der Körperflüssigkeit, beispielsweise die Blutglukosekonzentration, können über die Schnittstelle 178 an die Auswerteeinheit 168 kommuniziert werden, um dort zum Abgleich mit den Informationen des Sensorchips 118 des Okularsensors 110 genutzt zu werden. Somit kann das Messergebnis der Blutglukosemessung direkt in den Algorithmus der indirekten Blutzuckerbestimmung mit dem Okularsensor 110 eingeführt werden.

**Bezugszeichenliste**

| | | | |
|---|---|---|---|
| 110 | Okularsensor | 182 | Display |
| 112 | Augenseite | 184 | Teststreifen |
| 114 | Außenseite | 186 | Blutstropfen |
| 116 | Tageslicht | | |
| 118 | Sensorchip | | |
| 120 | Trägermaterial | | |
| 122 | Messdiode | | |
| 124 | Referenzdiode | | |
| 126 | Hintergrunddiode | | |
| 128 | Hintergrundfilter | | |
| 130 | Sensorfilter | | |
| 132 | Referenzfilter | | |
| 134 | Transmission Hintergrundfilter | | |
| 136 | Transmission Sensorfilter | | |
| 138 | Transmission Referenzfilter | | |
| 140 | Intensität Tageslicht | | |
| 142 | Intensität Anregungslicht | | |
| 144 | Gesamtfluoreszenz | | |
| 146 | Sensorfluoreszenz | | |
| 148 | Referenzfluoreszenz | | |
| 150 | Lichtfalle | | |
| 152 | Stege | | |
| 154 | Anregungseinheit | | |
| 156 | kapazitives Element | | |
| 158 | kapazitives Element | | |
| 160 | Widerstand | | |
| 162 | Widerstand | | |
| 164 | Generator | | |
| 166 | Messsystem | | |
| 168 | Auswerteeinheit | | |
| 170 | Mikrocomputer | | |
| 172 | Bedienelemente | | |
| 174 | Display | | |
| 176 | Kalibrationssystem | | |
| 178 | Schnittstelle | | |
| 180 | Bedienelemente | | |

## Patentansprüche

1. Okularsensor (110) zum Nachweis mindestens eines Analyten in einer Augenflüssigkeit, wobei der Okularsensor (110) mindestens ein Sensormaterial aufweist, wobei das Sensormaterial ausgestaltet ist, um bei Anwesenheit des mindestens einen Analyten mindestens eine optische Eigenschaft zu ändern, wobei der Okularsensor (110) weiterhin mindestens einen Sensorchip (118) aufweist, wobei der Sensorchip (118) mindestens einen integrierten optischen Detektor (122) zum Nachweis der optischen Eigenschaft aufweist, wobei der Okularsensor (110) ein Trägermaterial (120) aufweist, wobei der Sensorchip (118) in das Trägermaterial (120) eingebettet ist und wobei das Sensormaterial ganz oder teilweise in dem Trägermaterial (120) enthalten ist, wobei das Trägermaterial (120) ein für den Analyten zumindest teilweise durchlässiges Material umfasst, insbesondere ein Hydrogel, **gekennzeichnet dadurch, dass** das Sensormaterial auf mindestens eine der folgenden Weisen in dem Trägermaterial (120) enthalten ist: das Sensormaterial ist in das Trägermaterial (120) eingemischt; das Sensormaterial ist in dem Trägermaterial (120) gelöst; das Sensormaterial ist ganz oder teilweise Bestandteil des Trägermaterials (120), insbesondere in Form von funktionellen Gruppen, welche an ein Matrixmaterial des Trägermaterials (120) gebunden sind; das Sensormaterial ist in Mikrokapseln implementiert, insbesondere in Mikrokapseln, welche in das Trägermaterial (120) eindispergiert sind.

2. Okularsensor (110) gemäß dem vorhergehenden Anspruch, wobei das Trägermaterial (120) ein verformbares, insbesondere ein flexibles Material umfasst.

3. Okularsensor (110) gemäß einem der vorhergehenden Ansprüche, wobei die mindestens eine optische Eigenschaft eine durch ein Anregungslicht anregbare Lumineszenz umfasst, insbesondere eine Fluoreszenz oder eine Phosphoreszenz, wobei der Okularsensor (110) ausgestaltet ist, um eine Anregung des Sensormaterials durch eine Anregungslichtquelle zu ermöglichen.

4. Okularsensor (110) gemäß dem vorhergehenden Anspruch, wobei der Okularsensor (110) mindestens eine der folgenden Ausgestaltungen aufweist:
- der Okularsensor (110) ist ausgestaltet, um externem Anregungslicht (142), insbesondere Tageslicht (116), den Zugang zu dem Sensormaterial zu ermöglichen;
- der Sensorchip (118) weist eine integrierte Anregungslichtquelle, insbesondere eine integrierte Leuchtdiode und/oder eine Laserdiode, auf.

5. Okularsensor (110) gemäß einem der Ansprüche 3 bis 4, wobei der Okularsensor (110) weiterhin einen optischen Hintergrundfilter (128), insbesondere einen optischen Bandpassfilter oder Kantenfilter aufweist, welcher ausgestaltet und angeordnet ist, um das Licht der Anregungslichtquelle ganz oder teilweise zu filtern.

6. Okularsensor (110) gemäß einem der Ansprüche 3 bis 5, wobei der Okularsensor (110) weiterhin einen Sensorfilter (130) aufweist, insbesondere einen Bandpassfilter oder Kantenfilter, welcher ausgestaltet und angeordnet ist, um Lumineszenzlicht des Sensormaterials zu transmittieren und um Licht außerhalb des Wellenlängenbereichs des Lumineszenzlichts zumindest teilweise, vorzugsweise vollständig, zu unterdrücken.

7. Okularsensor (110) gemäß einem der Ansprüche 5 oder 6, wobei der Okularsensor (110) weiterhin ein Referenzmaterial aufweist, wobei das Referenzmaterial ausgestaltet ist, um mindestens eine optische Eigenschaft, insbesondere eine Lumineszenz, vorzugsweise eine Fluoreszenz oder eine Phosphoreszenz, in Abhängigkeit von der Intensität des Anregungslichts und unabhängig von der Anwesenheit des Analyten zu ändern.

8. Okularsensor (110) gemäß dem vorhergehenden Anspruch, wobei der Sensorchip (118) weiterhin einen optischen Referenzdetektor (124) aufweist, welcher ausgestaltet ist, um die optische Eigenschaft des Referenzmaterials zu messen.

9. Okularsensor (110) gemäß dem vorhergehenden Anspruch, wobei der Okularsensor (110) weiterhin einen Referenzfilter (132), insbesondere einen Bandpassfilter oder Kantenfilter, aufweist, wobei die optische Eigenschaft des Referenzmaterials eine Referenzlumineszenz ist und wobei der Referenzfilter (132) ausgestaltet und eingerichtet ist, um eine Transmission der Referenzlumineszenz zum Referenzdetektor (124) zu ermöglichen und eine Transmission von Licht mit einer Wellenlänge außerhalb des Wellenlängenbereichs der Referenzlumineszenz zu unterdrücken.

10. Okularsensor (110) gemäß einem der Ansprüche 3 bis 9, wobei der Okularsensor (110) weiterhin einen optischen Hintergrunddetektor (126) aufweist, welcher ausgestaltet ist, um eine Intensität des Anregungslichts (142) zu messen.

11. Okularsensor (110) gemäß einem der Ansprüche 5 bis 10, wobei der Okularsensor (110) weiterhin eine Lichtfalle (150) aufweist, wobei die Lichtfalle (150) ausgestaltet ist, um eine Transmission von Licht der Anregungslichtquelle zum optischen Detektor (122) zu unterdrücken und wobei die Lichtfalle (150) ausgestaltet ist, um eine Diffusion des Sensormaterials und/oder des Analyten zum optischen Detektor (122) zu ermöglichen.

12. Okularsensor (110) gemäß einem der vorhergehenden Ansprüche, wobei der Okularsensor (110) weiterhin eine Schnittstelle für den Austausch von Informationen mit einer Auswerteeinheit (168) aufweist, wobei die Schnittstelle mindestens eine Schnittstelle zur drahtlosen Datenübertragung aufweist.

13. Messsystem (166) zum Nachweis mindestens eines Analyten in einer Augenflüssigkeit, umfassend einen Okularsensor (110) gemäß einem der vorhergehenden Ansprüche, sowie weiterhin umfassend eine Auswerteeinheit (168), welche eingerichtet ist, um mit dem Sensorchip (118) Informationen auszutauschen.

14. Messsystem (166) gemäß dem vorhergehenden Anspruch, wobei die Auswerteeinheit (168) eine Anregungseinheit (154) umfasst, welche ausgestaltet ist, um mit einer Schnittstelle des Sensorchips (118) einen Schwingkreis zu bilden.

15. Messsystem (166) gemäß einem der Ansprüche 13 oder 14, weiterhin umfassend ein Kalibrationssystem (176), welches ausgestaltet ist, um mindestens eine Kalibrationsinformation über eine Konzentration des Analyten in der Augenflüssigkeit und/oder einer weiteren Körperflüssigkeit zu verarbeiten und eine Kalibration der Bestimmung der Analytkonzentration in der Augenflüssigkeit und/oder der weiteren Körperflüssigkeit durchzuführen.

## Claims

1. Ocular sensor (110) for verification of at least one analyte in an eye fluid, wherein the ocular sensor (110) has at least one sensor material, wherein the sensor material is designed to change at least one optical characteristic in the presence of the at least one analyte, wherein the ocular sensor (110) also has at least one sensor chip (118), wherein the sensor chip (118) has at least one integrated optical detector (122) for verification of the optical characteristic, wherein the ocular sensor (110) has a carrier material (120), wherein the sensor chip (118) is embedded in the carrier material (120), and wherein the sensor material is entirely or partially contained in the carrier material (120), wherein the carrier material (120) comprises a material which is at least partially permeable for the analyte, in particular a hydrogel, **characterized in that**, the sensor material is contained in the carrier material (120) in at least one of the following manners: the sensor material is mixed into the carrier material (120), the sensor material is dissolved in the carrier material (120); the sensor material is entirely or partially a component of the carrier material (120), in particular in the form of functional groups which are bound to a matrix material of the carrier material (120); the sensor material is implemented in microcapsules, in particular in microcapsules which are dispersed in the carrier material (120).

2. Ocular sensor (110) according to the preceding claim, wherein the carrier material (120) comprises a deformable, in particular a flexible, material.

3. Ocular sensor (110) according to any one of the preceding claims, wherein the at least one optical characteristic comprises luminescence which can be excited by excitation light, in particular a fluorescence or a phosphorescence, wherein the ocular sensor (110) is designed to allow excitation of the sensor material by an excitation light source.

4. Ocular sensor (110) according to the preceding claim, wherein the ocular sensor (110) has at least one of the following designs:
- the ocular sensor (110) is designed to allow external excitation light (142), in particular daylight (116), access to the sensor material,
- the sensor chip (118) has an integrated excitation light source, in particular an integrated light-emitting diode and/or a laser diode.

5. Ocular sensor (110) according to any one of claims 3 to 4, wherein the ocular sensor (110) also has an optical background filter (128), in particular an optical bandpass filter or edge filter, which is designed and arranged to entirely or partially filter the light from the excitation light source.

6. Ocular sensor (110) according to any one of claims 3 to 5, wherein the ocular sensor (110) also has a sensor filter (130), in particular a bandpass filter or edge filter, which is designed and arranged to transmit luminescence light of the sensor material and to at least partially, preferably completely, suppress light outside the wavelength range of the luminescence light.

7. Ocular sensor (110) according to any one of claims 5 and 6, wherein the ocular sensor (110) furthermore has a reference material, wherein the reference material is designed to change at least one optical characteristic, in particular a luminescence, preferably a fluorescence or a phosphorescence, as a function of the intensity of the excitation light and independently of the presence of the analyte.

8. Ocular sensor (110) according to the preceding claim, wherein the sensor chip (118) furthermore has an optical reference detector (124) which is designed to measure the optical characteristic of the reference material.

9. Ocular sensor (110) according to the preceding claim, wherein the ocular sensor (110) furthermore has a reference filter (132), in particular a bandpass filter or edge filter, wherein the optical characteristic of the reference material is a reference luminescence and wherein the reference filter (132) is arranged and designed to allow a transmission of the reference luminescence to the reference detector (124) and to suppress a transmission of light at a wavelength outside the wavelength range of the reference luminescence.

10. Ocular sensor (110) according to any one of claims 3 to 9, wherein the ocular sensor (110) furthermore has an optical background detector (126) which is designed to measure an intensity of the excitation light (142).

11. Ocular sensor (110) according to any one of claims 5 to 10, wherein the ocular sensor (110) furthermore has a light trap (150), wherein the light trap (150) is designed to suppress a transmission of light from the excitation light source to the optical detector (122), and wherein the light trap (150) is designed to allow diffusion of the sensor material and/or of the analyte to the optical detector (122).

12. Ocular sensor (110) according to any one of the preceding claims, wherein the ocular sensor (110) furthermore has an interface for interchanging information with an evaluation unit (168), wherein the interface has at least one interface for wire-free data transmission.

13. Measurement system (166) for verification of at least one analyte in an eye fluid, comprising an ocular sensor (110) according to any one of the preceding claims, furthermore also comprising an evaluation unit (168) which is designed to interchange information with the sensor chip (118) .

14. Measurement system (166) according to the preceding claim, wherein the evaluation unit (168) comprises an excitation unit (154) which is designed to form a resonant circuit with an interface of the sensor chip (118).

15. Measurement system (166) according to any one of claims 13 and 14, furthermore comprising a calibration system (176) which is designed to process at least one calibration information item relating to a concentration of the analyte in the eye fluid and/or a further bodily fluid, and to carry out a calibration of the determination of the analyte concentration in the eye fluid and/or the further bodily fluid.

## Revendications

1. Capteur oculaire (110) destiné à détecter au moins un analyte dans un fluide oculaire, dans lequel le capteur oculaire (110) comprend au moins un matériau de capteur, dans lequel le matériau de capteur est conçu pour qu'au moins une propriété optique soit modifiée en présence d'au moins un analyte, dans lequel le capteur oculaire (110) comprend en outre au moins une puce de capteur (118), dans lequel la puce de capteur (118) comprend au moins un détecteur optique intégré (122) destiné à détecter la propriété optique, dans lequel le capteur oculaire (110) comprend un matériau de support (120), dans lequel la puce de capteur (118) est incorporée dans le matériau de support (120) et dans lequel le matériau de capteur est contenu entièrement ou partiellement dans le matériau de support (120), dans lequel le matériau de support (120) comprend un matériau au moins partiellement perméable à l'analyte, en particulier un hydrogel, **caractérisé en ce que** le matériau de capteur est contenu dans le matériau de support (120) d'au moins l'une des manières suivantes: le matériau de capteur est mélangé au matériau de support (120); le matériau de capteur est dissous dans le matériau de support (120); le matériau de capteur fait partie entièrement ou partiellement du matériau de support (120), en particulier sous la forme de groupes fonctionnels qui sont liés à un matériau de matrice du matériau de support (120); le matériau de capteur est mis en oeuvre dans des microcapsules, en particulier dans des microcapsules qui sont dispersées dans le matériau de support (120).

2. Capteur oculaire (110) selon la revendication précédente, dans lequel le matériau de support (120) comprend un matériau déformable, en particulier un matériau flexible.

3. Capteur oculaire (110) selon l'une des revendications précédentes, dans lequel ladite au moins une propriété optique comprend une luminescence pouvant être excitée par une lumière d'excitation, en particulier une fluorescence ou une phosphorescence, dans lequel le capteur oculaire (110) est conçu pour permettre l'excitation du matériau de capteur par une source de lumière d'excitation.

4. Capteur oculaire (110) selon la revendication précédente, dans lequel le capteur oculaire (110) présente au moins l'une des configurations suivantes:
- le capteur oculaire (110) est conçu pour permettre à de la lumière d'excitation externe (142), en particulier de la lumière du jour (116), d'accéder au matériau de capteur;
- la puce de capteur (118) comporte une source de lumière d'excitation intégrée, en particulier une diode électroluminescente intégrée et/ou une diode laser.

5. Capteur oculaire (110) selon l'une des revendications 3 à 4, dans lequel ledit capteur oculaire (110) comporte en outre un filtre d'arrière-plan optique (128), en particulier un filtre passe-bande optique ou un filtre de bord, qui est conçu et agencé pour filtrer tout ou partie de la lumière de la source de lumière d'excitation.

6. Capteur oculaire (110) selon l'une des revendications 3 à 5, dans lequel le capteur oculaire (110) comporte en outre un filtre de capteur (130), en particulier un filtre passe-bande ou un filtre à arêtes, qui est conçu et agencé pour transmettre la lumière de luminescence du matériau de capteur et pour supprimer au moins partiellement, de préférence complètement, la lumière se situant en dehors de la gamme de longueurs d'onde de la lumière de luminescence.

7. Capteur oculaire (110) selon la revendication 5 ou 6, dans lequel le capteur oculaire (110) comporte en outre un matériau de référence, dans lequel le matériau de référence est conçu pour qu'au moins une propriété optique soit modifiée, en particulier une luminescence, de préférence une fluorescence ou une phosphorescence, en fonction de l'intensité de la lumière d'excitation et indépendamment de la présence de l'analyte.

8. Capteur oculaire (110) selon la revendication précédente, dans lequel la puce de capteur (118) comporte en outre un détecteur de référence optique (124) conçu pour mesurer la propriété optique du matériau de référence.

9. Capteur oculaire (110) selon la revendication précédente, dans lequel le capteur oculaire (110) comporte en outre un filtre de référence (132), en particulier un filtre passe-bande ou un filtre à arêtes, dans lequel la propriété optique du matériau de référence est une luminescence de référence et dans lequel le filtre de référence (132) est conçu et agencé pour permettre une transmission de la luminescence de référence vers le détecteur de référence (124) et pour supprimer une transmission d'une lumière ayant une longueur d'onde se situant en dehors de la gamme de longueurs d'onde de la luminescence de référence.

10. Capteur oculaire (110) selon l'une des revendications 3 à 9, dans lequel le capteur oculaire (110) comprend en outre un détecteur optique d'arrière-plan (126) conçu pour mesurer une intensité de la lumière d'excitation (142).

11. Capteur oculaire (110) selon l'une des revendications 5 à 10, dans lequel le capteur oculaire (110) comprend en outre un piège à lumière (150), dans lequel le piège à lumière (150) est conçu pour supprimer une transmission de la lumière de la source de lumière d'excitation vers le détecteur optique (122) et dans lequel le piège à lumière (150) est conçu pour permettre la diffusion du matériau de capteur et/ou de l'analyte vers le détecteur optique (122).

12. Capteur oculaire (110) selon l'une des revendications précédentes, dans lequel le capteur oculaire (110) comporte en outre une interface pour l'échange d'informations avec une unité d'évaluation (168), dans lequel l'interface comporte au moins une interface pour la transmission de données sans fil.

13. Système de mesure (166) destiné à détecter au moins un analyte dans un fluide oculaire, comprenant un capteur oculaire (110) selon l'une des revendications précédentes, et comprenant en outre une unité d'évaluation (168) conçue pour échanger des informations avec la puce de capteur (118).

14. Système de mesure (166) selon la revendication précédente, dans lequel l'unité d'évaluation (168) comprend une unité d'excitation (154) configurée pour former un circuit oscillant avec une interface de la puce de capteur (118).

15. Système de mesure (166) selon l'une des revendications 13 ou 14, comprenant en outre un système d'étalonnage (176) conçu pour traiter au moins une information d'étalonnage concernant une concentration de l'analyte dans le fluide oculaire et/ou dans un autre fluide corporel et pour effectuer un étalonnage de la détermination de la concentration de l'analyte dans le fluide oculaire et/ou dans l'autre fluide corporel.
